(19) Europäisches Patentamt

European Patent Office

Office européen des brevets

(11) **EP 1 466 646 A1**

(12) **DEMANDE DE BREVET EUROPEEN**

(43) Date de publication:
**13.10.2004 Bulletin 2004/42**

(51) Int Cl.⁷: **A61M 39/28**

(21) Numéro de dépôt: **03405252.2**

(22) Date de dépôt: **10.04.2003**

(84) Etats contractants désignés:
**AT BE BG CH CY CZ DE DK EE ES FI FR GB GR
HU IE IT LI LU MC NL PT RO SE SI SK TR**
Etats d'extension désignés:
**AL LT LV MK**

(71) Demandeur: **Nutricia Healthcare S.A.
1618 Chatel ST.Denis (CH)**

(72) Inventeurs:
 • **Devaud, Didier
 1110 Morges (CH)**
 • **Hazard, Jean-Marc
 1800 Vevey (CH)**

 • **Brosy, Philippe
 1652 Botterens (CH)**
 • **Rochat, Jean-Denis
 1272 Genolier (CH)**
 • **Hauswirth, Serge
 1163 Etoy (CH)**
 • **Magnenat, Olivier
 1110 Morges (CH)**

(74) Mandataire: **Savatier, Yves et al
Moinas & Savoye SA,
42, rue Plantamour
1201 Geneve (CH)**

(54) **Dispositif de sécurité contre l'écoulement libre**

(57) L'invention concerne un dispositif de sécurité pour arrêter l'écoulement libre d'un liquide dans un conduit souple (2) qui comporte des moyens d'écrasement (7a,7b) du conduit souple (2), caractérisé en ce que ces moyens d'écrasement (7a,7b) du conduit souple sont des moyens élastiques calibrés (6a,6b) pour ne permettre le passage du liquide que lorsque la pression du liquide dépasse une valeur déterminée.

Fig. 4

**Description**

La présente invention concerne un nouveau dispositif de sécurité pour arrêter l'écoulement libre d'un liquide dans un conduit souple.

Il est connu d'administrer à des patients des liquides ou fluides, notamment des solutions salines, de formulations aqueuses nutritives ou médicamenteuses par voie entérale (c'est-à-dire par l'intermédiaire du système digestif, par exemple par injection dans l'estomac) ou par voie parentérale (évitant le système digestif, par exemple par injection i.v.). Ces liquides sont en général fournis aux patients à partir d'un réservoir surélevé par rapport au patient par l'intermédiaire d'un conduit souple, le débit étant régulé soit uniquement au moyen d'une pince à galet (roller clamp) lorsque l'écoulement se fait par gravité, soit au moyen d'une pompe d'infusion, souvent une pompe péristaltique, qui dose l'injection au cours du temps.

Dans le cas de l'utilisation d'une pompe péristaltique il existe un risque lié au positionnement du conduit souple dans la tête de la pompe. Si celui-ci est mal placé ou s'il sort de son logement, le débit n'est plus contrôlé et le liquide peut s'écouler librement par gravité, avec parfois des conséquences très graves pour le patient, par exemple le surdosage d'un médicament ou la suralimentation avec un débordement de l'estomac vers les voies respiratoires. Ce risque existe également lors d'une administration par gravité dans le cas où l'utilisateur oublie de fermer la pince à galet.

Pour remédier au risque d'écoulement libre, diverses solutions ont été proposées.

Le brevet américain No. 6,461,335 ou No. 6,494,864 par exemple décrit une valve placée à l'intérieur du conduit souple agencée pour s'ouvrir automatiquement sous l'effet de la force de traction transmise à la partie du conduit souple en amont de la valve par la pression du liquide lorsque la pompe fonctionne correctement, et se refermer dès que cette pression est inférieure à un certain seuil. Le positionnement de cette valve à l'intérieur du conduit souple n'est pas facile à réaliser dans des conditions stériles.

Le brevet américain No. 4,689,043 propose un dispositif à positionner autour du conduit souple comportant des moyens d'écrasement de celui-ci, qui sont actionnables par une poignée de façon à obturer le conduit lorsque la pompe ne fonctionne pas correctement. Ce dispositif est de structure compliquée et n'est pas automatisé.

Le brevet américain No. 5,423,759 décrit un autre dispositif qui comporte des moyens d'écrasement du conduit souple, agencés pour obturer le conduit dès que la pression du liquide est inférieure à une valeur donnée, et un capteur de pression électronique qui mesure cette pression en amont du dispositif, la compare à une valeur de référence et envoie des signaux pour piloter les moyens d'écrasement. Ce dispositif comporte une partie électronique susceptible de dérèglements et coûteuse.

Le problème ou but de l'invention est de proposer un dispositif de sécurité contre l'écoulement libre qui ne présente pas les inconvénients sus-mentionnés.

Ce problème est résolu par l'invention telle que définie par le jeu de revendications ci-joint.

L'invention concerne un dispositif de sécurité pour arrêter l'écoulement libre d'un liquide dans un conduit souple qui comporte des moyens d'écrasement du conduit souple, caractérisé en ce que ces moyens d'écrasement du conduit souple sont des moyens élastiques calibrés pour ne permettre le passage du liquide que lorsque la pression du liquide dépasse une valeur donnée.

Selon le mode d'exécution préféré de l'invention, les moyens élastiques calibrés comprennent une paire de bras élastiques, chacun de ces bras portant une mâchoire d'écrasement et une surface d'appui. Ces bras élastiques sont soumis à une précontrainte initiale de flexion suffisante pour que les mâchoires d'écrasement obturent le conduit souple. Ces surfaces d'appui situées près de l'entrée du dispositif et en contact avec le conduit souple transmettent au bras élastiques une force proportionnelle à la pression du liquide en amont du dispositif. Les mâchoires d'écrasement ne s'ouvrent que lorsque cette force dépasse la précontrainte initiale de flexion des bras. On choisit cette précontrainte de façon à ne permettre cette ouverture que lorsque la pression du liquide dépasse une valeur seuil donnée, comprise entre la pression d'écoulement libre et la pression du liquide lorsque la pompe fonctionne correctement. Ainsi le dispositif arrêtera le liquide en cas d'écoulement libre et le laissera passer lorsque la pompe fonctionne correctement.

La précontrainte initiale de flexion des bras dépend de leur géométrie et des propriétés, en particulier du module d'élasticité de la matière qui les constitue. On dispose de formules permettant de calculer les dimensions des bras pour une matière donnée.

Le dispositif peut être constitué d'une matière plastique telle que le polypropylène, le polyéthylène de haute densité, le polystyrène, le chlorure de polyvinyl, un copolymère styrène-butadiène ou un copolymère acrylonitrile-butadiène-styrène. Il est alors commodément fabriqué par moulage par injection.

Le dispositif peut aussi être constitué d'un métal tel que l'aluminium ou l'acier inoxydable, en particulier l'acier à ressort inoxydable.

Il peut notamment être fabriqué à partir d'un fil d'acier inoxydable à ressort, ou encore être formé ou plié à partir d'une tôle ou d'une feuille métallique.

De préférence le dispositif de sécurité comporte des éléments de préhension pour agir manuellement sur les mâchoires d'écrasement de façon à permettre temporairement le passage du liquide, par exemple pour permettre l'amorçage de la ligne d'administration.

Selon un mode d'exécution avantageux le dispositif comporte des moyens pour maîtriser de façon précise l'ouverture des mâchoires d'écrasement de façon à réguler le débit du liquide. Ces moyens peuvent comprendre par exemple un doigt entraîné par une coulisse qui s'insère entre les mâchoires d'écrasement. Le même dispositif pourra alors servir soit à réguler le débit de liquide, de façon analogue à une pince à galet (roller clamp), lorsqu'on n'utilise pas de pompe, soit à protéger contre l'écoulement libre lorsqu'on utilise une pompe.

Le dispositif de sécurité de l'invention peut être utilisé dans toutes les applications où un risque existe en cas de passage de liquide au dessous d'une valeur donnée de la pression.

L'utilisation du dispositif de l'invention présente un intérêt particulier dans le domaine médical en particulier pour l'administration à des patients de solutions salines glucosées, de formulations aqueuses nutritives ou médicamenteuses, de solution saline ou glucosée, ou de solution isotonique par voie entérale ou parentérale.

Son utilisation est également avantageuse dans le domaine vétérinaire, l'agriculture, la chimie, le traitement de l'eau potable etc...

D'autres particularités et avantages de la présente invention apparaîtront dans la description qui va suivre, qui sera faite à l'aide des desseins annexés qui illustrent, schématiquement et à titre d'exemple, deux formes d'exécution du dispositif de sécurité contre l'écoulement libre de cette invention.

La figure 1 est un schéma d'une installation de distribution d'un liquide à un patient dans lequel le dispositif selon l'invention est inséré;

la figure 2 représente les principales forces et les paramètres importants pour calculer les dimensions des bras de flexion élastique du dispositif de l'invention;

la figure 3 est une vue en perspective éclatée de la première forme d'exécution;

la figure 4 est une vue en coupe longitudinale en élévation du dispositif de la figure 3 assemblé;

la figure 5 une vue en perspective de dessus de la deuxième forme d'exécution;

la figure 6 est une vue en perspective de dessous de la forme d'exécution de la figure 5.

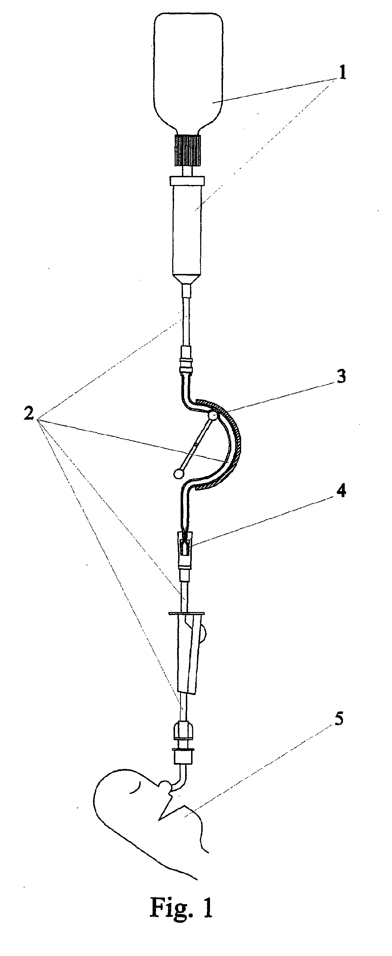

La figure 1 montre un réservoir de liquide 1 surélevé duquel sort un conduit souple 2 dont un segment passe dans une pompe péristaltique 3 qui envoie un débit contrôlé de liquide vers le patient 5 et dont un autre segment passe dans le dispositif de sécurité selon l'invention 4 situé entre la pompe 3 et le patient 5.

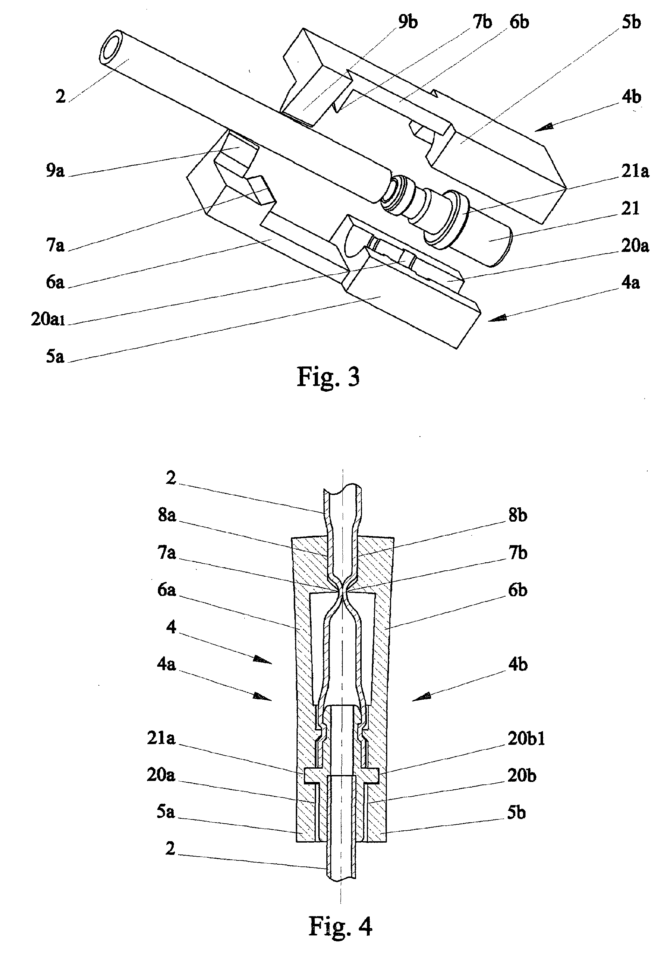

Le dispositif de sécurité 4 représenté sur les figures 3 et 4 comprend deux moitiés sensiblement symétriques 4a, 4b, qui comportent chacune un corps parallélépipédique 5a, 5b. Ces corps 5a, 5b sont assemblés comme illustré par la figure 4 et fixés l'un à l'autre par des moyens appropriés tels que par soudage, clipsage ou vissage par exemple. Chaque corps parallélépipédique 5a, 5b est solidaire d'un bras élastique 6a, 6b portant à son extrémité une mâchoire de section sensiblement triangulaire 7a, 7b définissant une ligne d'écrasement transversale au conduit souple 2. Chaque bras élastique présente encore une surface d'appui 8a, 8b destinée à déformer la section du conduit souple 2 de manière à avoir une grande surface de contact entre ce conduit souple 2 et les surfaces d'appui 8a, 8b, pour permettre une bonne transmission de la pression du conduit souple 2 aux bras élastiques 6a, 6b. Deux éléments de préhension 9a, 9b s'étendent le long de deux bords latéraux opposés respectifs des surfaces d'appui 8a, 8b.

Chaque corps parallélépipédique 5a, 5b comporte un évidement semi-cylindrique 20a, 20b dont le profil longitudinal est sensiblement complémentaire de celui d'un raccord 21 destiné à réunir deux segments du conduit souple 2. Ce raccord 21 présente une collerette annulaire 21a destinée à être logée dans une portion correspondante $20a_1$, $20b_1$ des évidements semi-cylindriques 20a, 20b pour former un ancrage entre le dispositif de sécurité et le conduit souple 2.

Comme illustré par la figure 4, lorsque le dispositif est monté et traversé par le conduit souple 2 et que les deux mâchoires 7a, 7b écrasent ce conduit souple 2, les deux bras flexibles 6a, 6b sont soumis à une précontrainte initiale de flexion, suffisante pour obturer le conduit souple 2 lorsque la pression du liquide dans ce conduit souple en amont des mâchoires 7a, 7b (en haut sur la figure 4) ne dépasse pas une valeur seuil correspondant à la pression de l'écoulement libre du liquide dans ce conduit souple 2.

Lorsque la pompe fonctionne correctement, la pression en amont du dispositif de sécurité est transmise aux bras élastiques 6a, 6b, par l'intermédiaire du conduit souple 2 en contact avec les surfaces d'appui 8a, 8b. Cette pression doit développer une force suffisante pour écarter les mâchoires 7a, 7b et ouvrir le conduit souple afin de laisser passer le liquide.

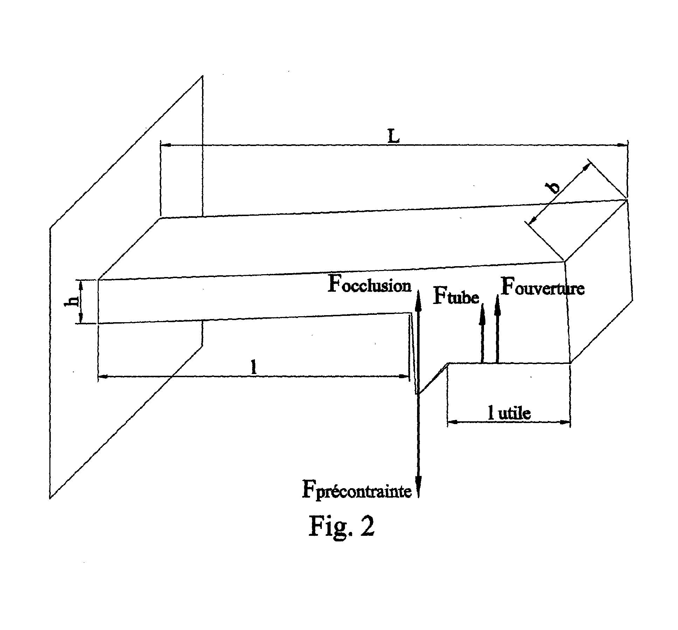

Les dimensions des bras élastiques 6a, 6b peuvent être calculées à partir des équations suivantes. Les principales forces et autres paramètres importants pour ce calcul sont représentés sur la Figure 2.

$$\sum M_{ext} = 0$$

$$F_{occlusion}.l + F_{tube}\cdot\left(L - \frac{l_{utile}}{2}\right) + F_{ouverture}\cdot\left(L - \frac{l_{utile}}{2}\right) - F_{précontrainte}.l = 0$$

$$\Rightarrow F_{précontrainte} = F_{occlusion} + \left(F_{tube} + F_{ouverture}\right).\frac{L - \frac{l_{utile}}{2}}{l}$$

dans laquelle

$F_{précontrainte}$ : force de tension initiale générée par la flexion du bras

$F_{occlusion}$ : force nécessaire pour assurer l'occlusion

$F_{tube}$ : force due au léger serrage appliqué sur le tube

$F_{ouverture}$ : force due à la pression appliquée sur la surface plane

L : longueur totale de la partie mobile

1 : longueur du bras flexible

$l_{utile}$ : longueur de la surface qui permet l'ouverture

Les dimensions du bras flexible sont calculées à l'aide de l'équation suivante :

$$F_{prétension} = \frac{3.E.I.f}{l^3}$$

dans laquelle $I = b.h^3/12$, b étant la largeur du bras et h étant son épaisseur

E est le module d'élasticité et f est la flexion (déplacement vers le haut le long de l'axe $F_{PR}$-$F_{OC}$).

Les éléments de préhension 9a, 9b permettent, si nécessaire, d'écarter manuellement les mâchoires 7a,7b de façon à ouvrir le dispositif de sécurité momentanément et de manière réversible, par exemple pour chasser l'air lors de l'amorçage de la ligne d'administration du liquide (priming).

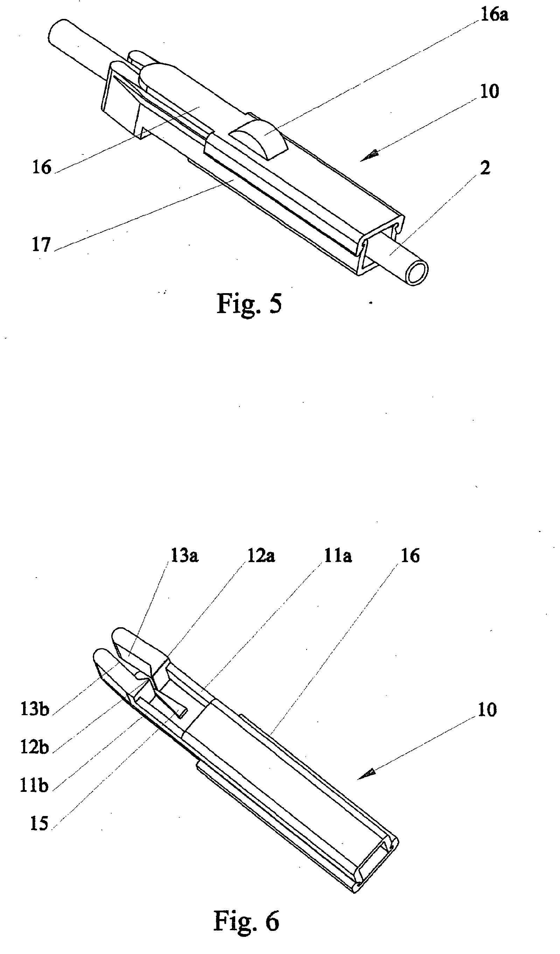

Le dispositif 10 selon la seconde forme d'exécution représentée par les figures 5 et 6 comporte un corps creux allongé 17 de section rectangulaire en forme de U, destiné à recevoir le conduit souple 2. Ce corps creux allongé 17 se termine à une extrémité par deux bras élastique 11a, 11b qui présentent une paire de mâchoires 12a, 12b, de section sensiblement triangulaire, définissant chacune une ligne d'écrasement transversale au conduit souple 2. Une paire de surfaces d'appui 13a, 13b s'étendent au-delà des mâchoires 12a, 12b. Ces surfaces d'appui 13a, 13b sont destinée à déformer légèrement la section du conduit souple 2 de manière à avoir une grande surface de contact entre ce conduit souple 2 et ces surfaces d'appui 13a, 13b, pour permettre une bonne transmission de la pression du conduit souple 2 aux bras élastiques 11a, 11b.

Une coulisse 16 comportant une saillie de préhension 16a est montée coulissante sur le corps allongé 17 et ferme l'ouverture de sa section en U. Cette coulisse 16 porte un doigt sensiblement triangulaire 15 susceptible de s'insérer entre les mâchoires 12a, 12b lors du déplacement de la coulisse 16 en direction des mâchoires 12a, 12b de manière à les écarter manuellement.

Ce dispositif peut s'utiliser soit pour régler avec précision le débit du liquide à l'aide de la coulisse 16 et du doigt triangulaire 15, de façon analogue aux pinces à roulette (roller clamps) utilisées de façon conventionnelle, soit pour arrêter l'écoulement libre en fonction de la pression exercée par le conduit souple 2 par l'intermédiaire des surfaces d'appui 13a, 13b.

Dans une variante, on pourrait soit conformer les mâchoires 12a, 12b de manière à ce qu'elles forment entre elles un très léger angle de quelques degrés, de préférence 2° ou 3°, qui permette la fermeture du conduit 2, mais permet de l'ouvrir plus à une extrémité de la section qu'à l'autre extrémité de cette même section. Ceci permet d'assurer un très faible écoulement. Ce résultat pourrait aussi être obtenu par exemple en conformant les faces latérales du doigt triangulaire 15 de manière à induire une légère torsion lorsqu'il agit sur les bras élastiques 11a, 11b.

Dans le cas d'une utilisation de cette seconde forme d'exécution en tant que dispositif de sécurité pour prévenir l'écoulement intempestif du liquide, le doigt 15 n'est pas inséré entre les mâchoires 12a, 12b et le dispositif fonctionne de façon très semblable au dispositif 4 selon la première forme d'exécution.

Dans une variante non représentée le dispositif de sécurité selon l'invention pourrait comporter un seul bras élastique 6a ou 6b, 11a ou 11b.

En variante de la première forme d'exécution illustrée par les figures 3 et 4, les bras élastiques peuvent être soit déformables manuellement au-delà de la limite élastique, soit cassables manuellement pour supprimer la fonction de sécurité du dispositif, au cas où on voudrait utiliser la tubulure à laquelle le dispositif de sécurité est associé avec un écoulement par gravité et non par une pompe.

Le dispositif de sécurité décrit dans les deux formes d'exécution est représenté pour fonctionner dans un sens d'écoulement dans le conduit 2. On pourrait aussi imaginer une variante où il fonctionnerait dans les deux sens en disposant une seconde paire de surfaces d'appui 8a, 8b, 13a, 13b de l'autre côté des mâchoires 7a, 7b, respectivement 12a, 12b. La surface de ces secondes surfaces d'appui pourrait être différente, de manière à ce que le conduit 2 s'ouvre à des pressions différentes suivant le sens de l'écoulement dans ce conduit 2.

## Revendications

1.  Dispositif de sécurité pour arrêter l'écoulement libre d'un liquide dans un conduit souple (2) qui comporte des moyens d'écrasement (7a, 7b ;12a, 12b) du conduit souple (2), **caractérisé en ce que** ces moyens d'écrasement (7a, 7b; 12a, 12b) du conduit souple sont des moyens élastiques calibrés (6a, 6b ;11a, 11b) pour ne permettre le passage du liquide que lorsque la pression du liquide dépasse une valeur déterminée.

2.  Dispositif selon la revendication 1, **caractérisé en ce que** les moyens élastiques calibrés (6a,6b ;11a,11b) comprennent au moins un bras élastique portant une mâchoire d'écrasement (7a, 7b; 12a, 12b) et au moins une surface d'appui (8a, 8b; 13a, 13b) disposée en amont desdits moyens d'écrasement et conformée pour provoquer une certaine déformation du conduit souple (2) pour assurer la transmission de la pression entre ledit conduit souple et ledit bras élastique.

3.  Dispositif selon la revendication 1, **caractérisé en ce que** les moyens élastiques calibrés (6a,6b ;11a,11b) comprennent au moins un bras élastique portant une mâchoire d'écrasement (7a, 7b; 12a, 12b) et au moins deux surfaces d'appui disposées de part et d'autre de laditemâchoire d'écrasement et conformées pour provoquer une certaine déformation du conduit souple (2) pour assurer la transmission de la pression entre ledit conduit souple et ledit bras élastique.

4.  Dispositif selon la revendication 3, **caractérisé en ce que** lesdites deux surfaces d'appui ont des surfaces différentes de manière à ce que le conduit souple (2) s'ouvre à des pressions différentes suivant le sens de l'écoulement dans ce conduit.

5.  Dispositif selon l'une des revendications précédentes, **caractérisé en ce que** le bras élastique (6a, 6b) comporte un élément de préhension (9a, 9b) pour agir manuellement sur les mâchoires d'écrasement de façon à permettre le passage du liquide temporairement.

6.  Dispositif selon l'une des revendications précédentes, **caractérisé en ce qu'**il comporte de plus des moyens (15, 16) pour commander l'ouverture manuelle des mâchoires d'écrasement (12a, 12b).

7.  Dispositif selon la revendication 6, **caractérisé en ce que** les moyens (15, 16) pour commander l'ouverture manuelle des mâchoires d'écrasement (12a, 12b) comprennent un doigt (15) sensiblement triangulaire solidaire d'une coulisse (16) dont le déplacement sert à régler la pénétration dudit doigt triangulaire entre lesdites mâchoires d'écrasement.

8.  Dispositif selon l'une des revendications précédentes, **caractérisé en ce qu'**il est constitué d'une matière plastique telle que le polypropylène, le polyéthylène de haute densité, le polystyrène, le chlorure de polyvinyle, un copolymère styrène-butadiène ou un copolymère acrylonitrile-butadiène-styrène.

9.  Dispositif selon l'une des revendications précédentes, **caractérisé en ce qu'**il est constitué d'un métal tel que l'aluminium ou l'acier inoxydable.

10.  Utilisation du dispositif selon l'une des revendications précédentes pour l'administration entérale ou parentérale d'une formulation nutritive.

11.  Utilisation du dispositif selon l'une des revendications précédentes pour l'administration entérale ou parentérale d'une formulation médicamenteuse, de solution saline ou glucosée, ou de solution isotonique.

Fig. 1

Fig. 2

Fig. 3

Fig. 4

Fig. 5

Fig. 6

Office européen
des brevets

**RAPPORT DE RECHERCHE EUROPEENNE**

Numéro de la demande

EP 03 40 5252

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 6 454 742 B1 (FOURNIE GLENN G ET AL) 24 septembre 2002 (2002-09-24) * colonne 1, ligne 14 - ligne 22; figures 1-10 * * colonne 6, ligne 48 - colonne 7, ligne 2 * * colonne 7, ligne 16 - ligne 63 * * revendication 7 * --- | 1-5,8, 10,11 | A61M39/28 |
| X | EP 0 423 978 A (MINNESOTA MINING & MFG) 24 avril 1991 (1991-04-24) * colonne 2, ligne 3 - ligne 32 * * colonne 3, ligne 46 - colonne 4, ligne 48 * * figures 1-6 * --- | 1,2,5-7 | |
| X | US 6 142 979 A (HALBERT AL ET AL) 7 novembre 2000 (2000-11-07) * colonne 1, ligne 54 - ligne 61; figures 1-8 * * colonne 2, ligne 1 - ligne 27 * * colonne 4, ligne 46 - ligne 63 * * colonne 5, ligne 13 - ligne 31 * * colonne 10, ligne 13 - ligne 17 * --- | 1-3,9 | |
| X | US 5 154 704 A (ARCHIBALD G KENT) 13 octobre 1992 (1992-10-13) * colonne 1, ligne 23 - ligne 26; figures 1-4 * * colonne 2, ligne 60 - ligne 66 * * colonne 3, ligne 25 - colonne 4, ligne 15 * * colonne 5, ligne 59 - colonne 6, ligne 9 * * colonne 6, ligne 27 - ligne 31 * ----- | 1,2,5 | |

**DOMAINES TECHNIQUES RECHERCHES (Int.Cl.7)**

A61M
F16K

Le présent rapport a été établi pour toutes les revendications

| Lieu de la recherche | Date d'achèvement de la recherche | Examinateur |
|---|---|---|
| MUNICH | 11 septembre 2003 | Reinbold, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................
& : membre de la même famille, document correspondant

EPO FORM 1503 03.82 (P04C02)

EP 1 466 646 A1

## ANNEXE AU RAPPORT DE RECHERCHE EUROPEENNE
## RELATIF A LA DEMANDE DE BREVET EUROPEEN NO.

EP 03 40 5252

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-09-2003

| Document brevet cité au rapport de recherche | | Date de publication | Membre(s) de la famille de brevet(s) | | Date de publication |
|---|---|---|---|---|---|
| US 6454742 | B1 | 24-09-2002 | AUCUN | | |
| EP 0423978 | A | 24-04-1991 | US | 5017192 A | 21-05-1991 |
| | | | AU | 626460 B2 | 30-07-1992 |
| | | | AU | 6303390 A | 26-04-1991 |
| | | | CA | 2025519 A1 | 21-04-1991 |
| | | | DE | 69016347 D1 | 09-03-1995 |
| | | | DE | 69016347 T2 | 10-08-1995 |
| | | | EP | 0423978 A2 | 24-04-1991 |
| | | | HK | 1006287 A1 | 19-02-1999 |
| | | | JP | 3140164 A | 14-06-1991 |
| US 6142979 | A | 07-11-2000 | US | 5810323 A | 22-09-1998 |
| | | | AU | 4406999 A | 10-04-2000 |
| | | | WO | 0016827 A1 | 30-03-2000 |
| US 5154704 | A | 13-10-1992 | AUCUN | | |

EPO FORM P0460